# EUROPEAN PATENT APPLICATION

(11) **EP 4 512 419 A1**
(43) Date of publication of application: **26.02.2025**
(21) Application number: 23791262.1
(22) Date of filing: 19.04.2023
(51) Int. Cl.: A61K 39/116, A61K 39/385, A61P 31/04

(54) **POLYVALENT PNEUMOCOCCAL POLYSACCHARIDE CONJUGATE VACCINE COMPONENT AND APPLICATION THEREOF**

(30) Priority: 19.04.2022 CN 202210411982
(71) Applicant: Shanghai Reinovax Biologics Co., Ltd, Shanghai 201210 (CN); Shanghai Microdom Biotech Co., Ltd, Shanghai 201413 (CN)
(72) Inventor: ZHU, Xianchao, Shanghai 201203 (CN); CHEN, Huagen, Shanghai 201203 (CN); XIONG, Xishuang, Shanghai 201203 (CN); LIU, Chang, Shanghai 201203 (CN); LI, Ying, Shanghai 201203 (CN); XIA, Qingfeng, Shanghai 201203 (CN); HE, Ping, Shanghai 201203 (CN); WANG, Juanjuan, Shanghai 201203 (CN); HUANG, Xiaomin, Shanghai 201203 (CN)
(74) Representative: Nederlandsch Octrooibureau
(86) International application number: PCT/CN2023/089161
(87) International publication number: WO 2023/202607

(57) **Abstract**

The present invention relates to a polyvalent pneumococcal polysaccharide protein conjugate and immunogenicity thereof, and specifically provides an immunogenic composition containing capsular polysaccharides of streptococcus pneumoniae from different serotypes, and a carrier, the serotypes at least comprising 2, 8, 9N, 10A, 11A, 12F, 15B, 17F, 20, 22F and 33F. The immunogenic composition can improve the immunogenicity of polysaccharides of different serotypes, and may prevent invasive infection caused by pneumococci of various different serotypes.

## Description

### FIELD OF THE INVENTION

The present description relates to the field of biomedicine, and in particular to the prevention of infection by bacterial pathogens through multivalent vaccine immunization.

### BACKGROUND OF THE INVENTION

Streptococcus pneumoniae (S.pneumoniae) is a capsulated Gram-positive diplococcus.Streptococcus pneumoniae can be divided into nearly 100 serotypes according to difference in capsular polysaccharide. Capsular polysaccharide is an important pathogenic factor. Streptococcus pneumoniae normally parasitizes in the nasopharynx of healthy people. When the parasitic environment changes, such as decreased body resistance, respiratory viral infections such as measles and influenza, or malnutrition, older or infirmed adults, streptococcus pneumoniae can penetrate through the mucosal defense system and cause invasive infections, such as entering the lower respiratory tract to cause pneumonia, crossing the blood-brain barrier to cause bacterial meningitis, passing through alveolar epithelial cells, invading vascular endothelial cells and entering the blood to cause bacteremia, and it can also migrate from the nasopharynx into the sinuses, causing sinusitis, and enter the middle ear through the eustachian tube, causing otitis media to non-invasively spread to other parts of the respiratory tract.

Pneumococcal disease is one of the serious public health problems worldwide. According to the World Health Organization, in 2005, about 1.6 million people died of pneumococcal disease every year in the world, including 700,000 to 1,000,000 children under the age of 5, most of whom lived in developing countries. It can be seen that pneumococcus has been seriously endangering the health of children. In developed countries, pneumococcal disease mainly occurs in children under 2 years of age and the elderly, as well as immunocompromised persons of all age groups.

According to the infection sites of pneumococcal, pneumococcal diseases can be divided into two categories: invasive pneumococcal disease (IPD) and non-invasive pneumococcal disease (NIPD). The common treatment is antibiotic therapy. However, pneumococcal resistance to commonly used antimicrobial drugs has become a growing problem globally. Years of clinical practice have proven that pneumococcal vaccination is the most cost-effective way to prevent pneumococcal disease.

Launched pneumonia vaccines include pneumococcal polysaccharide vaccine (PPSV) and pneumococcal polysaccharide protein conjugate vaccine (PCV). The 23-valent pneumonia polysaccharide vaccine (PPSV23) is not suitable for infants and people with low immunity due to its low immunogenicity and lack of immune memory and boosting effects. Pneumonia polysaccharide conjugate vaccines that have launched and can be used in infants and young children include PCV7, PCV10 and PCV13, but their serum types are few, their immune protection coverage is low, and they may cause immunosuppression. For example, a literature meta-analysis on the distribution of Streptococcus pneumoniae serotypes in China continental showed that the coverage rate of PCV10 serotypes from 2000 to 2016 was 52.3%, and the coverage rate of PCV13 serotypes was 68.4%. In addition, the incidence of non-vaccine serotype diseases has also been found to increase after vaccination. In addition, since the carrier protein used in polysaccharide-protein conjugate vaccines is also widely used in immunization of infants and young children, immunosuppression caused by high doses or repeated use of carrier proteins is also a potential risk in the clinical application of polysaccharide-protein conjugate vaccines.

Therefore, the serotype coverage rate of existing polysaccharide-protein conjugate vaccines is not ideal. It is clinically significant to develop a multivalent pneumonia conjugate vaccine to cover a wider range of pathogenic serotypes of pneumococci, increase non-vaccine serotypes, improve the coverage protection and immunogenicity of the new multivalent vaccines, and reduce the risk of immunosuppressive effects of carrier proteins.

### SUMMARY OF THE INVENTION

In order to solve the problems of poor immunogenicity of pneumococcal polysaccharide vaccine, low serum coverage of polysaccharide conjugate vaccine, low immunogenicity, and immunosuppression, the present description provides a multivalent immunogenicity composition with wider application, stronger immunogenicity and weakened immunosuppression.

A first aspect of the present description provides an immunogenic composition containing capsular polysaccharides from different serotypes of Streptococcus pneumonia and a vehicle, the serotypes includes at least 2, 8, 9N, 10A, 11A, 12F, 15B, 17F, 20, 22F and 33F.

In one or more embodiments, the serotypes include 8, 9, 10, 11, 12, 13, 14, 15, 16, 17, 18, 19, 20, 21, 22, 23, 24 serotypes selected from the following: serotype 1, 2, 3, 4, 5, 6A, 6B, 7F, 8, 9N, 9V, 10A, 11A, 12F, 14, 15B, 17F, 18C, 19A, 19F, 20, 22F, 23F and 33F, and the serotypes include at least serotype 2, 8, 9N, 10A, 11A, 12F, 15B, 17F, 20, 22F and 33F. Preferably, the serotypes include the following 20 serotypes: serotype 1, 3, 4, 5, 6A, 6B, 7F, 8, 9V, 10A, 11A, 12F, 14, 15B, 18C, 19A, 19F, 22F, 23F, 33F, or the following 24 serotypes: serotype 1, 2, 3, 4, 5, 6A, 6B, 7F, 8, 9N, 9V, 10A, 11A, 12F, 14, 15B, 17F, 18C, 19A, 19F, 20, 22F, 23F and 33F.

In one or more embodiments, the vehicle is one or more selected from the goup consisting of: saline, Ringer's solution and phosphate buffered saline.

In one or more embodiments, the immunogenic composition further comprises an adjuvant.

In one or more embodiments, the adjuvant is an aluminum-based adjuvant.

In one or more embodiments, the adjuvant includes one or more selected from the group consisting of aluminum phosphate, aluminum sulfate, aluminum hydroxide, monophosphoryl lipid A, QS21, CpG, MF59, stearoyltyrosine, Freund's adjuvant, and other mucosal adjuvants.

In one or more embodiments, in the composition, the weight ratio of the capsular polysaccharide from serotype 3, 6B or 12F to any other one capsular polysaccharide is 10:1 to 1:10, such as 5:1 to 1:5, preferably 2:1.

In one or more embodiments, the composition is a preparation, and the concentrations of capsular polysaccharides from serotypes 3, 6B and 12F are each independently 1 to 8 µg/dose (preferably 4 µg/dose), and the concentrations of the remaining capsular polysaccharides are each independently 0.5 to 5 µg/dose (preferably 2 µg/dose). Further, the concentration of aluminum phosphate adjuvant is 0.125 mg/dose to 0.5 mg/dose.

In one or more embodiments, the composition further includes a surfactant, such as Tween 20 or Tween 80. Preferably, the concentration of the surfactant in the composition is 100-300 µg/dose, preferably 70-120 µg/dose.

The present description also provides a multivalent immunogenic composition comprising a plurality of polysaccharide-protein conjugates and pharmaceutically acceptable excipients, wherein each polysaccharide-protein conjugate contains capsular polysaccharide from different serotypes of Streptococcus pneumonia conjugated to a carrier protein.

In one or more embodiments, the carrier protein includes at least two carrier proteins.

In one or more embodiments, the carrier protein includes (1) CRM197 and (2) TTD or a variant thereof.

In one or more embodiments, the TTD is the C-terminal domain of TT.

In one or more embodiments, the TTD has the sequence shown in SEQ ID NO: 2 and the TTD variant has a sequence that is at least 90% sequence identical to SEQ ID NO: 2.

In one or more embodiments, the serotypes include at least serotype 2, 8, 9N, 10A, 11A, 12F, 15B, 17F, 20, 22F, and 33F. Preferably, the serotypes include the following 20 serotypes: serotype 1, 3, 4, 5, 6A, 6B, 7F, 8, 9V, 10A, 11A, 12F, 14, 15B, 18C, 19A, 19F, 22F, 23F, 33F, or the following 24 serotypes: serotype 1, 2, 3, 4, 5, 6A, 6B, 7F, 8, 9N, 9V, 10A, 11A, 12F, 14, 15B, 17F, 18C, 19A, 19F, 20, 22F, 23F and 33F.

In one or more embodiments, in the polysaccharide protein conjugate, at least one or more or all capsular polysaccharides from serotypes 3, 5, 6B, 12F, 15B, 18C, 19F and 23F, respectively conjugated to the carrier protein TTD or a variant thereof.

In one or more embodiments, the serotypes include 20 serotypes in which the capsular polysaccharides of 8, 9, 10, 11, 12, 13, 14, 15, 16, 17, 18, 19 or 20 serotypes, including serotype 3, 5, 6B, 12F, 15B, 18C, 19F and 23F, are respectively conjugated to the carrier protein TTD or a variant thereof.

In one or more embodiments, the serotypes include 20 serotypes, wherein the capsular polysaccharides of serotype 3, 5, 6B, 12F, 15B, 18C, 19F and 23F are respectively conjugated to the carrier protein TTD or a variant thereof; wherein the capsular polysaccharides of serotypes 1, 4, 6A, 7F, 8, 9V, 10A, 11A, 14, 19A, 22F and 23F are respectively conjugated to the carrier protein CRM197.

In one or more embodiments, the serotypes include 24 serotypes in which the capsular polysaccharides of 8, 9, 10, 11, 12, 13, 14, 15, 16, 17, 18, 19, 20, 21, 22, 23 or 24 serotypes, including serotype 3, 5, 6B, 12F, 15B, 18C, 19F and 23F, are respectively conjugated to the carrier protein TTD or a variant thereof.

In one or more embodiments, the serotypes include 24 serotypes, wherein the capsular polysaccharides of serotype 3, 5, 6A, 6B, 9N, 11A, 12F, 15B, 17F, 18C, 19A, 19F, 20, 23F and 33F are respectively conjugated to the carrier protein TTD or a variant thereof; wherein the capsular polysaccharides of serotype 1, 2, 4, 7F, 8, 9V, 10A, 14 and 22F are respectively conjugated to the carrier protein CRM197.

In one or more embodiments, the serotypes include 24 serotypes, wherein the capsular polysaccharides of serotypes 3, 5, 6B, 12F, 15B, 18C, 19F and 23F are respectively conjugated to the carrier protein TTD or a variant thereof; wherein the capsular polysaccharides of serotypes 1, 2, 4, 6A, 7F, 8, 9N, 9V, 10A, 11A, 14, 17F, 19A, 20, 22F, and 33F are respectively conjugated to the carrier protein CRM197.

In one or more embodiments, the weight ratio of the capsular polysaccharide from serotype 3, 6B or 12F to any other capsular polysaccharide is 10:1 to 1:10, such as 5:1 to 1:5, preferably 2:1.

In one or more embodiments, the composition further comprises an adjuvant, such as an aluminum-based adjuvant.

In one or more embodiments, the adjuvant is selected from aluminum phosphate, aluminum sulfate and aluminum hydroxide, preferably aluminum phosphate.

In one or more embodiments, the weight ratio of conjugate to adjuvant in the composition is from 1:10 to 1:2, preferably from 54:500 to 54:125.

In one or more embodiments, the composition is a preparation, and the concentrations of capsular polysaccharides from serotypes 3, 6B and 12F are each independently 1 to 8 µg/dose (preferably 4 µg/dose), and the concentrations of the remaining capsular polysaccharides are each independently 0.5 to 5 µg/dose (preferably 2 µg/dose), and the concentration of the aluminum phosphate adjuvant is 0.125 mg/dose to 0.5 mg/dose.

In one or more embodiments, the composition further includes a surfactant, such as Tween 20 or Tween 80. Preferably, the concentration of the surfactant in the composition is 100-300 µg/dose, preferably 70-120 µg/dose.

In one or more embodiments, the pH of the composition is 5.0 to 7.0, preferably 5.0 to 6.2.

The present description also provides the use of the immunogenic composition according to the first aspect of the present description in the preparation of a medicament for inducing an immune response to a pneumococcal capsular polysaccharide conjugate and/or an immune response to tetanus toxin.

In one or more embodiments, the medicament is used to prevent or treat pneumococcal infection and/or tetanus toxin infection.

The invention also provides a method of inducing an immune response to a pneumococcal capsular polysaccharide conjugate and/or an immune response to tetanus toxin, comprising administering to a subject an immunologically effective amount of the immunogenic composition according to the first aspect of the description.

The present description also provides an immune composition that results in passive immunity, comprising a bactericidal antibody targeting pneumococcis, the antibody being obtained by immunizing a mammal with the immunogenic composition according to any embodiment of the invention. In one or more embodiments, the bactericidal antibodies are present in serum, gamma globulin fractions or purified antibody preparations.

### Advantages of the invention:

The immunogenic composition of the present description can effectively prevent invasive infections of 24 different serotypes of pneumococcal bacteria, and can induce relatively balanced and high immunogenicity for 24 serotypes, with the immunogenicity being related to the ratio of the conjugate content of each serotype. The immunogenic composition of the present description comprises different carrier proteins at the same time, and both carrier proteins are non-toxic, so there is no need for detoxification treatment. This dual-carrier design reduces potential safety and immunosuppressive risks. The immunogenic composition is also protective against infection with tetanus toxin.

### Description of drawings

Figure 1, comparison of the immunogenicity of type 5 pneumococcal polysaccharide conjugates of different carrier.
Figure 2, comparison of the immunogenicity of 24-valent pneumococcal polysaccharide conjugate vaccines in mice at different adjuvant doses (the same 13 serotypes as the positive vaccine).
Figure 3, comparison of the immunogenicity of 24-valent pneumococcal polysaccharide conjugate vaccines in mice at different adjuvant doses (11 serotypes other than the 13 serotypes of the positive vaccine).
Figure 4, comparison of the immunogenicity of 24-valent pneumococcal polysaccharide conjugate vaccines at different pH in mice.
Figure 5, comparison of the immunogenicity of the same 13 valents serotype in mice (D35).
Figure 6, comparison of polysaccharide antibody titers of serotypes other than PCV13 vaccine in mice (D35).
Figure 7, comparison of titers of polysaccharide antibodies of the same 13-valent serotype in rabbits (D35).
Figure 8, comparison of polysaccharide antibody titers of serotypes other than the 13 serotypes in mice (D35).
Figure 9, serum antibody titer levels after two doses vaccination of dual-carrier and single-carrier 24-valent pneumonia polysaccharide conjugate vaccines, respectively.
Figure 10, comparison of serum titers after two doses vaccination of PCV20 dual-carrier and single-carrier vaccines, respectively.
Figure 11, comparison of serum titers after two doses vaccination of PCV24 dual-carrier and single-carrier vaccines, respectively.
Figure 12, survival curve of protective efficacy of 24-valent pneumococcal polysaccharide conjugate vaccine against type 3 pneumococcal challenge in mice.
Figure 13, survival curve of protective efficacy of 24-valent pneumococcal polysaccharide conjugate vaccine against type 22F pneumococcal challenge in mice.
Figure 14, comparison of TT antibody titers after the second dose of immunization and before immunization. Group A is normal saline; group B is diphtheria-tetanus pertussis vaccine; group C is 24-valent pneumococcal polysaccharide conjugate vaccine.
Figure 15, survival rate curve after tetanus toxin challenge.

### DETAILED DESCRIPTION

The present description provides novel 20-valent or 24-valent pneumococcal capsular polysaccharide-protein conjugates and corresponding immunogenic compositions and vaccine preparations. Compared with the launched 13-valent polysaccharide conjugate vaccine on the market, the vaccine preparation of the present description induces higher antibody titers on some serotypes; it also shows good immunogenicity to serotypes other than the 13 serotypes; and it can induce better immune protection in vivo compared with the 13-valent polysaccharide conjugate vaccine and the 23-valent polysaccharide vaccine. In addition, the inventors also found that using different carriers on different conjugates can induce higher antibody titers than the single-carrier vaccine group.

The present description first provides an immunogenic composition for inducing immune response of mammals to pneumococcal capsular polysaccharide conjugates and protecting mammals from pneumococcal infection. The immunogenic composition containing capsular polysaccharides from different serotypes of Streptococcus pneumonia, including at least serotype 2, 8, 9N, 10A, 11A, 12F, 15B, 17F, 20, 22F and 33F. Preferably, the serotypes include: serotype 1, 2, 3, 4, 5, 6A, 6B, 7F, 8, 9N, 9V, 10A, 11A, 12F, 14, 15B, 17F, 18C, 19A, 19F, 20, 22F, 23F and 33F.

The pneumococcal capsular polysaccharide present in the immunogenic composition can be in the form of a free polysaccharide or as a component of a conjugate in which the polysaccharide is covalently linked to a protein. In addition to the polysaccharide or polysaccharide-protein conjugate, the immunogenic composition may also comprise a pharmaceutically acceptable vehicle, such as saline, Ringer's solution, or phosphate-buffered saline.

In a preferred embodiment of the present description, the capsular polysaccharide is covalently linked to the protein to form a conjugate. Thus, any protein or fragment thereof that is acceptable to an individual and which is capable of inducing an immune cell (e.g. T-cell) dependent response is suitable for conjugation to the pneumococcal capsular polysaccharide. Basically any protein can serve as a conjugating protein. In particular, the selected protein must have at least one free amino group for conjugation to the polysaccharide. Preferably the protein is any natural or recombinant bacterial protein and is itself an immunogen that induces a T-cell dependent response in young and adult mammals. Examples of such proteins include, but are not limited to, tetanus toxoid, cholera toxin, diphtheria toxoid, and CRM197 or variants thereof. Other candidates for conjugation proteins include toxins or toxoids of Pseudomonas, Staphylococcus, Streptococcus, Pertussis, and enterotoxigenic bacteria including E. coli.

In a preferred embodiment of the present description, the pneumococcal capsular polysaccharide is covalently linked to the protein to form a conjugate. Thus, any protein or fragment thereof that is acceptable to an individual and which is capable of inducing an immune cell (e.g. T-cell) dependent response is suitable for conjugation to the pneumococcal capsular polysaccharide. Basically any protein can serve as a conjugating protein. In particular, the selected protein must have at least one free amino group for conjugation to the polysaccharide. Preferably the protein is any natural or recombinant bacterial protein and is itself an immunogen that induces a T-cell dependent response in young and adult mammals. Examples of such proteins include, but are not limited to, tetanus toxoid, cholera toxin and diphtheria toxoid. Other candidates for conjugation proteins include toxins or toxoids of Pseudomonas, Staphylococcus, Streptococcus, Pertussis, and enterotoxigenic bacteria including E. coli.

The protein conjugated to the pneumococcal capsular polysaccharide (i.e., the carrier protein) may be any protein or fragment thereof that is acceptable to an individual and capable of inducing an immune cell (eg, T-cell) dependent response. Basically any protein can serve as a conjugating protein. In particular, the selected protein must have at least one free amino group for conjugation to the polysaccharide. Preferably the protein is any natural or recombinant bacterial protein and is itself an immunogen that induces a T-cell dependent response in young and adult mammals. Examples of such proteins include, but are not limited to, tetanus toxoid, cholera toxin and diphtheria toxoid. Other candidates for conjugation proteins include toxins or toxoids of Pseudomonas, Staphylococcus, Streptococcus, Pertussis, and enterotoxigenic bacteria including E. coli.

Alternatively, non-toxic variants of protein toxins, such as CRM197, may be used. Preferably such mutations retain epitopes of the native toxin. These mutated toxins are called "cross-reactive materials" or CRMs. The CRM197 carrier protein (NCBI: AMV91693.1, SEQ ID NO: 3) is a non-toxic variant of diphtheria toxoid. The protein is non-toxic but retains the immunogenicity of diphtheria toxoid (DT). Its fermentation and purification methods are available in published articles and patents (US5614382). The non-toxic variant protein of diphtheria toxoid (CRM197) is a carrier protein that has been clinically proven to be safe and effective, and has been widely used in marketed pneumococcal polysaccharide conjugate vaccines. Other diphtheria toxin variants are also suitable for use as carrier proteins. It is also possible to conjugate pneumococcal polysaccharides with fragments of these proteins, provided that these fragments should be sufficiently long, i.e. preferably at least 10 amino acids to define T-cell epitopes.

Tetanus toxoid protein (TT) has been reported extensively in the open literature. The inventors discovered a variant of the TT protein, the TTD protein. The protein is non-toxic but retains the immunogenicity of the TT protein. The TTD protein is located at the C-terminus of the heavy chain of the TT protein, with a relative molecular weight of 50 KDa. It is the receptor binding region of the toxin, has no toxicity, has good immunogenicity, and is less allergenic than tetanus toxoid. It is a potential tetanus vaccine antigen component and carrier protein. TTD can be obtained by recombinant expression and purification (Immunobiology, Vol. 216, Issue 4, 2011, P 485-490). An exemplary TTD expression and purification method includes: cloning a DNA sequence expressing TTD (SEQ ID NO: 1) into a protein **expression** plasmid pET21, and constructing an engineered bacterium (e.g., Escherichia coli BL21 (DE3)) that recombinantly expresses the TTD protein (SEQ ID NO: 2); picking a recombinant engineering bacteria monoclonal colony and proliferting in a suitable culture medium and conditions (for example, BL21 (DE3) was cultured in 10 mL of LB (Amp) liquid medium at 37°C, 250 rpm until OD₆₀₀ reaches 0.8), adding an inducer for culture (for example, 0.1 mM IPTG was added and continued to culture at 25°C, 250 rpm for 4 hours), and then TTD was separated from the culture. The process of isolating proteins from culture is well known in the art, for example, comprising the following steps: the culture medium was centrifuged at 8000 rpm and 4°C, and then the cells were broken (e.g., ultrasonically broken) after being resuspended in PBS, and the broken liquid was centrifuged at 8000 rpm and 4°C to collect the supernatant containing TTD protein. TTD protein can be purified from the supernatant by the method commonly used in the art to purify proteins from liquids, such as ammonium sulfate precipitation, clarification filtration, combined chromatography such as ion exchange chromatography, composite medium chromatography, and/or affinity chromatography, etc., with a purity of more than 95%. The molecular weight determined by mass spectrometry was consistent with the theoretical molecular weight.

Other suitable carrier proteins include inactivated bacterial toxins, such as tetanus toxoid, pertussis toxoid, cholera toxoid (e.g., WO 2004/083251), E. coli LT, E. coli ST, and exotoxin from Pseudomonas aeruginosa. Bacterial outer membrane proteins, such as outer membrane complex c (OMPC), porins, transferrin binding protein, pneumolysin, pneumococcal surface protein A (PspA), pneumococcal adhesin protein (PsaA), C5a peptidase from group A or group B Streptococci, or Haemophilus influenzae protein D can also be used. Other proteins such as ovalbumin, keyhole limpet hemocyanin (KLH), bovine serum albumin (BSA), or purified protein derivative (PPD) of tuberculin can also be used as carrier proteins.

Without substantially affecting the TTD activity (e.g., toxin binding activity), a person skilled in the art can change one or more (e.g., 1, 2, 3, 4, 5, 6, 7, 8, 9 or 10 or more) amino acids in the TTD of the present description to obtain a variant of TTD. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In the art, conservative substitution with amino acids with similar or similar properties usually does not change the function of the protein. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present description. The TTD variants described herein include TTD variants having at least 90% (e.g., at least 95%, at least 98%, at least 99%) sequence identity to the TTD shown in SEQ ID NO: 2 and retaining its toxin binding activity.

The inventors have found that the immunogenic composition formed by conjugating various serotype polysaccharides with different carrier proteins and mixing them has better immunogenicity and lower risk of immunosuppression. Exemplarily, the immunogenic composition comprises at least two carrier proteins, such as CRM197 and TTD or variants thereof. In a preferred embodiment, 20 or 24 pneumococcal polysaccharides of the present description are chemically coupled with CRM197 and TTD carrier proteins to prepare polysaccharide-protein conjugates. The obtained polysaccharide-protein conjugates of different serotypes have good physical and chemical properties, such as a polysaccharide-protein ratio between 0.5 and 3.0, a free sugar content below 20%, and other residual impurities are controlled within a very low range.

In some embodiments, in the polysaccharide protein conjugate, at least one or more or all capsular polysaccharides from serotype 3, 5, 6B, 12F, 15B, 18C, 19F and 23F, respectively conjugated to the carrier protein TTD or a variant thereof. Further, 8, 9, 10, 11, 12, 13, 14, 15, 16, 17, 18, 19, 20, 21, 22, 23 or 24 serotypes including serotype 3, 5, 6B, 12F, 15B, 18C, 19F and 23F are respectively conjugated to the carrier protein TTD or its variants. Preferably, the capsular polysaccharides of serotype 3, 5, 6B, 12F, 15B, 18C, 19F and 23F are respectively conjugated to the carrier protein TTD or a variant thereof; the capsular polysaccharides of serotype 1, 2, 4, 6A, 7F, 8, 9N, 9V, 10A, 11A, 14, 17F, 19A, 20, 22F, and 33F are respectively conjugated to the carrier protein CRM197.

Herein, in the polysaccharide-protein conjugate or immunogenic composition, the ratio of capsular polysaccharides from different serotypes to carrier proteins is between 0.2-3.0, for example 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0 or a range between any two of the above values, preferably 0.5-2.5, 0.5-2.0, 0.5-1.5, more preferably 0.6-2.5, 0.6-2.0, 0.6-1.5.

In the polysaccharide protein conjugate or immunogenic composition described herein, the weight ratio of the capsular polysaccharide from serotype 3, 6B or 12F to any other capsular polysaccharide is 10:1 to 1:10, such as 5:1 to 1:5, preferably 2:1. For example, the composition is a preparation, and the concentrations of capsular polysaccharides from serotypes 3, 6B and 12F are each independently 1 to 8 µg/dose (preferably 4 µg/dose), and the concentrations of the remaining capsular polysaccharides are each independently 0.5 to 5 µg/dose (preferably 2 µg/dose), and the concentration of the aluminum phosphate adjuvant is 0.125 mg/dose to 0.5 mg/dose.

The immunogenic composition can be used as a vaccine, further comprising an adjuvant such as an aluminum-based adjuvant (aluminum hydroxide, aluminum phosphate or aluminum sulfate). In addition, the immunogenic compositions described herein may further comprise a surfactant, such as Tween 20 or Tween 80. The immunogenic compositions of the present description are capable of inducing active and passive protection against pneumococcal infection. For passive protection, immune antibodies are produced by immunizing a mammal with a vaccine prepared from the immunogenic composition of the present description and then recovering the immune antibodies from the mammal. The invention also provides methods of immunizing a subject against pneumococcal infection by administering an immunologically effective amount of a composition of the invention.

Capsular polysaccharides are prepared by standard techniques known to those skilled in the art. In the present description, capsular polysaccharide is prepared from Streptococcus pneumoniae serotype 1, 3, 4, 5, 6A, 6B, 7F, 8, 9V, 9N, 10A, 11A, 12F, 14, 15B, 18C, 19A, 19F, 20, 22F, 23F and 33F. These pneumococcal conjugates are prepared by separate methods and formulated into single dose formulations.

Bacterial capsule polysaccharide is an important component of thallus and an important virulence factor of pathogenic bacteria. Pneumococcal bacterial capsular polysaccharides are released into the culture medium during bacterial culture. The pneumococcal capsular polysaccharide in the present description is obtained by culturing, fermenting and purifying pneumococcal bacteria of different serotypes. This type of technology is mature and has been used in the industry for many years, with multiple published articles or patent reports (US4242501). In one embodiment, each pneumococcal polysaccharide serotype is cultured in soy-based medium. Various polysaccharides are then purified by centrifugation, precipitation, ultrafiltration and column chromatography (US4686102).

A specific method for preparing capsular polysaccharide is specifically reported in US Patent US5714354. Pneumococcal bacterial culture\fermentation and polysaccharide preparation is a mature process (US4686102, US5847112). Briefly, different Streptococcus pneumonia strains were inoculated into a shake flask containing a culture medium (such as soybean culture medium), cultured in a 37°C carbon dioxide incubator overnight, inoculated the culture solution into a 10-liter fermentation tank, and fermented and cultured for 6-12 hours. After the culture was completed, DOC inactivator was added for sterilization and inactivation. The clarified culture solution was harvested by centrifugation, CTAB precipitant was added to the clarified culture solution to obtain crude precipitated complex sugars, and then through a series of precipitation, washing, and chromatography purification to obtain refined polysaccharides.

The purification method of pneumococcal capsular polysaccharide is also a common technique in the art, for example, it is accomplished by an organic solvent-free method (US Patent US5714354). Usually the purification process of refined polysaccharides includes, but is not limited to, NaCl solution was added to dissolve the complex sugar, and the above solution was centrifuged to take the supernatant, alcohol was added to remove impurities and precipitated sugar, the precipitate was washed multiple times to remove impurities, or the refined polysaccharides can be obtained by removing impurities or using chromatography methods such as ion exchange columns and composite packing purification methods according to different sugars. In a specific embodiment, the purification process mainly includes: the bacterial inactivated liquid was centrifuged to separate the thallus, the centrifugal supernatant was harvested, and then filtered through a 0.22 µm filter membrane and concentrated through 100 KD membrane cassette ultrafiltration to obtain the pneumonia polysaccharide fermentation concentrate. CTAB solution was added to the pneumonia polysaccharide fermentation concentrate, the above mixture was stirred at room temperature, centrifuged at 8000 rpm, and the polysaccharide complex precipitate was collected (for 7F, 14, and 33F polysaccharides, the supernatant was collected). The polysaccharide complex was then dissociated with 0.25-0.5M NaCl solution, and then purified through precipitation, column chromatography, ultrafiltration and other processes to obtain a purified polysaccharide solution. The polysaccharide solution was filtered through a 0.22 µm membrane and stored at low temperature.

Methods for quality analysis of the resulting polysaccharides are known in the art, for example by nuclear magnetic resonance. Specifically, the prepared different types of pneumococcal capsular polysaccharides can be used to identify serotypes through specific serum, the chemical structure of the pneumococcal polysaccharides can be analyzed through nuclear magnetic resonance (NMR) spectroscopy, and the content of functional groups (such as rhamnose, uronic acid, O-acetyl and other components) contained in polysaccharides can be analyzed through chemical color development methods. It was determined that the physical and chemical indicators of the polysaccharide meet the standards set by the European Pharmacopoeia for pneumococcal polysaccharides, and the contents of major impurities such as protein and nucleic acid are less than 1%.

A number of conjugation methods can be used to produce the polysaccharide-protein conjugates of the invention. During the polysaccharide-protein binding reaction, polysaccharide activation and protein conjugation steps are usually included. Chemical activation of the polysaccharide and subsequent conjugation to the carrier protein is achieved by conventional methods. See, for example, U.S. Patent US4673574 and US4902506. By controlling parameters such as the molecular weight of polysaccharide, polysaccharide activation reaction, polysaccharide-protein reaction ratio, reaction temperature, reaction time, and reaction solution pH value, the final polysaccharide-protein conjugate is obtained. The obtained polysaccharide-protein conjugate is further purified to remove unreacted substances and impurities, and its polysaccharide-protein cross-linking degree and residual impurities are quantified through mass analysis to ensure the consistency and comparability of batches.

The purified polysaccharide is chemically activated to enable the sugar to react with the carrier protein. According to the characteristics of polysaccharides, different chemical activation methods can be selected. Commonly used polysaccharide activation methods include cyanogen bromide method (US6375846B1), hydrolysis (US4761283), 1-cyano-4-dimethylaminopyridine tetrafluoroborate (CDAP) (EP0720485), periodic acid oxidation method (US4711779). The activated polysaccharides can be separated from other impurities by ultrafiltration. After activation, the polysaccharide has the activity to react with the carrier protein.

For example, selective oxidation of a periodate salt (eg, sodium periodate) or its equivalent is used to oxidize the hydroxyl group of the previous sugar moiety to form an aldehyde group. This forms activated pneumococcal capsular polysaccharide, which can now be covalently linked to a carrier protein of choice. For example, at room temperature, using about 1 ml of about 20 mM sodium periodate solution to suitably oxidize about 10 mg of polysaccharide for about 10-15 minutes; alternatively, adding 0.05 to 2 equivalents of periodate to the polysaccharide for 12-24 hours. The reaction time can be varied with other amounts of periodate to obtain equivalent oxidation. The reduction and ring-opening of sugars activates the ortho-hydroxyl groups of reducing sugars into aldehyde groups. After the polysaccharide activation is completed, the small molecular substances in the reaction can be removed by ultrafiltration and concentration.

The conjugate is prepared by chemically coupling the polysaccharide fragment obtained by processing the purified and extracted pneumococcal outer membrane polysaccharide to the carrier protein. The polysaccharide-protein conjugates can be obtained by direct chemical coupling or through a linker, such as adipate dihydrazide. Various chemical methods for conjugating polysaccharides to proteins are known and described in the literature. The common methods include cyanogen bromide method, CDAP method or reductive amination method (US5952454, EP0720485, US4711779). For example, US Patent 4,644,059, incorporated herein by reference, describes conjugates prepared using adipic acid dihydrazide (ADH) as the homobifunctional linker. U.S. Patent 4,695,624, also incorporated by reference herein, describes methods for preparing polysaccharides and preparing conjugates using intergeneric spacers. A general study of the various preparation methods and factors used to design conjugates is discussed in Dick, William E. and Michel Beurret, Contrib. Mrcrobil. Immunol. (1989), Vol. 10, pp. 48-114, which is also incorporated by reference. The polysaccharide-protein conjugate obtained by conjugation can induce relatively strong immunogenicity, and can simultaneously induce specific antibodies against polysaccharides and proteins.

A preferred method of conjugating the various pneumococcal capsular polysaccharide-protein conjugates of the invention is reductive amination. The activated pneumococcal capsular polysaccharide is conjugated to the conjugated protein of choice by coupling the amino group of the carrier protein to the aldehyde group of the pneumococcal capsular polysaccharide in the presence of cyanoborohydride ions or another reducing agent. The pneumococcal capsular polysaccharide-protein conjugates resulting from the reductive amination process are preferably soluble in aqueous solutions. This makes the pneumococcal capsular polysaccharide-protein conjugates of the invention a preferred candidate for use as a vaccine. The conjugation conditions can be adjusted according to the molecular weight of the polysaccharide and the specific protein. In the reductive amination method, the ratio of polysaccharide to carrier protein is controlled 0.5 to 3.0. During the reaction, 1.0 to 2.0 equivalents of sodium cyanoborohydride solution of the polysaccharide is added, and then 2.0 equivalents of sodium cyanoborohydride solution is added. The incubation temperature may be room temperature, and the incubation time may be, for example, at least 12 hours. After the reaction, use ultrafiltration to change the liquid or chromatography to remove impurities or unreacted substrates in the reaction. Finally, sterile filter through a 0.22um filter, and store the polysaccharide-protein conjugate solution at 2 to 8°C.

After capsular polysaccharide is conjugated to a carrier protein, the polysaccharide-protein conjugate is purified by a variety of techniques. These techniques include concentration/diafiltration operations, precipitation/elution and column chromatography.

After purifying each complex sugar, they are mixed to formulate the immunogenic composition of the invention, which can be used as a vaccine. Formulation of the immunogenic compositions of the present description can be accomplished using methods well known in the art. For example, 20 or 24 individual pneumococcal conjugates may be formulated with pharmaceutically acceptable excipients to prepare a composition. Examples of such vehicles include, but are not limited to, water, buffered saline, polyols (eg, glycerol, propylene glycol, liquid polyethylene glycol), and glucose solutions.

In some embodiments, immunogenic compositions can include one or more adjuvants. As defined herein, an "adjuvant" is a substance used to enhance the immunogenicity of the immunogenic composition of the invention. Thus, adjuvants are often administered to enhance immune responses and are well known to the skilled person. Suitable adjuvants for enhancing the effectiveness of the composition include, but are not limited to: (1) aluminum salts, such as aluminum hydroxide, aluminum phosphate, aluminum sulfate, etc.; (2) oil-in-water emulsion formulations, such as MF59, SAF, RibiTM Adjuvant System (RAS), (Corixa, Hamilton, MT); (3) saponin adjuvants, such as QuilA or STIMULONTMQS-21; (4) bacterial lipopolysaccharides (such as aminoalkyl glucosamine phosphate compounds (AGP) or its derivatives or analogs), synthetic polynucleotides (such as oligonucleotides containing CpG motifs); (5) cytokines, such as interleukins (e.g., IL-1, IL-2, IL-4. IL-5, IL-6, IL-7, IL-12, IL-15, IL-18, etc.), interferon (e.g., gamma interferon), granulocyte macrophage colony-stimulating factor (GM-CSF), macrophage colony-stimulating factor (M-CSF), tumor necrosis factor (TNF), co-stimulatory molecules B7-1 and B7-2, etc.; (6) mucosal adjuvants, including protein toxins, recombinant protein subunits bases and nucleic acids, including detoxified mutants of bacterial ADP-ribosylating toxins, such as wild-type or mutant cholera toxin (CT), pertussis toxin (PT), or E. coli heat-labile toxin (LT) (see For example, WO93/13302 and WO92/19265) and (7) other substances that act as immunostimulants to enhance the effectiveness of the composition. The dosage of the adjuvant can be determined by those skilled in the art according to routine techniques. Exemplarily, the concentration of adjuvant (eg, aluminum phosphate) in the immunogenic compositions herein ranges from 0.125 mg/dose to 0.5 mg/dose.

The immunogenic compositions and vaccines of the present description can be used to protect or treat mammals (eg, humans) susceptible to pneumococcal infection by administering the vaccine systemically or mucosally. The description also provide the use of the immunogenic compositions described herein for the preparation of a medicament for providing protection against a variety of pneumococcal infections.

The immunogenic compositions of the present description are useful as a means of increasing antibodies for prophylactic and diagnostic purposes. The diagnostics are particularly useful for monitoring and detecting various infections and diseases caused by pneumococcal bacteria. Another embodiment of the present description uses immunogenic compositions as immunogens for active and passive immunogenic protection of individuals at risk for pneumococcal infection or disease. Immunizing antibodies for passive protection are generated by immunizing a mammal with any of the immunogenic compositions of the invention and then recovering the bactericidal antibodies in the gamma-globulin fraction from the mammal, either as serum or as specific antibodies. The vaccines of the invention used herein are capable of inducing antibodies that provide protection against pneumococcal infection.

Additionally, the pneumococcal capsular polysaccharide itself can be used as an immunizing agent, preferably in combination with an adjuvant, such as an aluminum-based adjuvant, as an immunogenic composition. A further embodiment of the invention is the use of the immunogenic composition as immunogenic protection against pneumococcal infection.

The immunogenic compositions and vaccines of the present description may typically be formed by dispersing the pneumococcal capsular polysaccharide or conjugate into suitable pharmaceutically acceptable excipients. The auxiliary materials include, but are not limited to, diluents, vehicles, solubilizers, emulsifiers, preservatives and/or adjuvants. Excipients are preferably non-toxic to the recipient at the dosage and concentration employed, for example: saline, buffer, glucose, water, glycerol, ethanol and combinations thereof. In some embodiments, the composition may contain substances for improving, maintaining, or retaining, for example, the pH, permeability, viscosity, clarity, color, isotonicity, odor, sterility, stability, dissolution or release rate, absorption, or permeation of the composition. These substances are known in the prior art. The optimal pharmaceutical composition can be determined according to the expected route of administration, mode of delivery and required dose. Additives commonly used in vaccines may also be present, for example stabilizers such as lactose or sorbitol and adjuvants such as aluminum phosphate, aluminum hydroxide, aluminum sulfate, CpG, monophosphoryl lipid A, QS21, MF59 or stearoyltyrosine.

The protective or therapeutic effects of the immunogenic compositions and vaccines of the present description can be accomplished by administering the vaccine via systemic or mucosal routes. Such administration includes parenteral administration, such as injection by the intramuscular, intraperitoneal, intradermal or subcutaneous route, or transmucosal administration to the oral/alimentary, respiratory or genitourinary tracts. In one embodiment, intranasal administration is used to treat pneumonia or otitis media.

The dosage of the immunogenic composition should be such that an immunogenic effect is achieved. The amount of conjugate per vaccine dose is selected as the amount that induces immune protection without significant adverse effects. This amount may vary depending on the pneumococcal serotype. Typically, each dose will contain 0.01 µg to 100 µg of polysaccharide or conjugate, for example 0.1 µg to 10 µg, or 1 µg to 5 µg. The administered doses generally range from about 0.01 µg to about 10 µg per kilogram of body weight. A range of optimal immunizing doses can be given. A unit dosage form of the vaccine may contain an equivalent amount of about 0.01 µg to about 100 µg of the pneumococcal capsular polysaccharide or conjugate, for example, 0.1 µg to 10 µg, or 1 µg to 5 µg.

The optimal amounts of the components of a particular vaccine are determined by standard studies involving observation of appropriate immune responses in subjects. After the initial vaccination, subjects may receive one or several booster immunizations spaced sufficiently intervals. The immunogenic compositions of the invention are suitable for use by infants, children, adolescents and adults. The immunization regimen can be determined by experienced clinicians.

In a specific embodiment of the invention, the 20 or 24 valent vaccine is sterile liquid preparation of pneumococcal capsular polysaccharides of serotypes 1, 2, 4, 6A, 7F, 8, 9N, 9V, 10A, 11A, 14, 17F, 19A, 20, 22F and 33F respectively conjugated to CRM197, and pneumococcal capsular polysaccharides of serotypes 3, 5, 6B, 12F, 15B, 18C, 19F and 23F respectively conjugated to TTD. Each dose is formulated to contain: 2 µg of each polysaccharides except 4 µg of polysaccharide 3, 6B, or 12F; approximately 20 to 80 µg of CRM197 carrier protein; approximately 10 to 40 µg of TTD carrier protein; 0.125 mg to 0.5 mg of aluminum phosphate adjuvant. The liquid is filled into a single-dose syringe without preservatives. After shaking, the vaccine is homogeneous and the white suspension is ready for intramuscular administration.

Exemplary embodiments of the present application:
1. An immunogenic composition containing capsular polysaccharides from different serotypes of Streptococcus pneumonia and a vehicle, the serotypes includes at least 2, 8, 9N, 10A, 11A, 12F, 15B, 17F, 20, 22F and 33F,
   preferably, the serotypes include 8, 9, 10, 11, 12, 13, 14, 15, 16, 17, 18, 19, 20, 21, 22, 23, 24 serotypes selected from the following: 1 , 2, 3, 4, 5, 6A, 6B, 7F, 8, 9N, 9V, 10A, 11A, 12F, 14, 15B, 17F, 18C, 19A, 19F, 20, 22F, 23F and 33F, and the serotypes include at least 2, 8, 9N, 10A, 11A, 12F, 15B, 17F, 20, 22F and 33F.
2. The immunogenic composition of item 1, wherein the serotypes include the following 20 serotypes: 1, 3, 4, 5, 6A, 6B, 7F, 8, 9V, 10A, 11A, 12F, 14, 15B, 18C, 19A, 19F, 22F, 23F, 33F, or the following 24 serotypes: 1, 2, 3, 4, 5, 6A, 6B, 7F, 8, 9N, 9V, 10A, 11A , 12F, 14, 15B, 17F, 18C, 19A, 19F, 20, 22F, 23F and 33F,
   preferably, the vehicle is selected from one or more of the group consiting of: saline, Ringer's solution and phosphate buffered saline.
3. The immunogenic composition of item 1 or 2, wherein the immunogenic composition further contains an adjuvant,
   preferably, the adjuvant includes one or more selected from the group consisting of: aluminum-based adjuvants, monophosphoryl lipid A, QS21, CpG, MF59, stearoyltyrosine, Freund's adjuvant and other mucosal adjuvants.
4. The immunogenic composition of item 1 or 2, wherein the weight ratio of the capsular polysaccharide from serotype 3, 6B or 12F to any other one capsular polysaccharide is 10:1 to 1:10, for example, 5:1 to 1:5,
   preferably, the composition is a preparation, and the concentrations of capsular polysaccharides from serotypes 3, 6B and 12F are each independently 1 to 8 µg/dose, and the concentrations of the remaining capsular polysaccharides are each independently 0.5 to 5 µg/dose.
5. A multivalent immunogenic composition comprising a plurality of polysaccharide-protein conjugates and pharmaceutically acceptable excipients, wherein each polysaccharide-protein conjugate contains capsular polysaccharide from different serotypes of Streptococcus pneumonia conjugated to a carrier protein,
   preferably, the carrier protein contains at least two carrier proteins,
   preferably, the serotypes include at least 2, 8, 9N, 10A, 11A, 12F, 15B, 17F, 20, 22F and 33F; more preferably, the serotypes include the following 20 serotypes: 1, 3, 4, 5, 6A, 6B, 7F, 8, 9V, 10A, 11A, 12F, 14, 15B, 18C, 19A, 19F, 22F, 23F, 33F, or the following 24 serotypes: 1, 2, 3, 4 , 5, 6A, 6B, 7F, 8, 9N, 9V, 10A, 11A, 12F, 14, 15B, 17F, 18C, 19A, 19F, 20, 22F, 23F and 33F.
6. The multivalent immunogenic composition of item 5, wherein the carrier protein includes (1) CRM197 and (2) TTD or a variant thereof,
   preferably, the TTD is the C-terminal domain of TT,
   More preferably, the TTD has the sequence shown in SEQ ID NO.2, and the TTD variant has a sequence with at least 90% sequence identity to SEQ ID NO:2.
7. The multivalent immunogenic composition of item 5 or 6, wherein, for the polysaccharide protein conjugate, at least one or more or all of the capsular polysaccharides from serotypes 3, 5, 6B, 12F, 15B, 18C, 19F and 23F are respectively conjugated to the carrier protein TTD or a variant thereof,
   preferably,
   the serotypes include 20 serotypes in which the capsular polysaccharides of 8, 9, 10, 11, 12, 13, 14, 15, 16, 17, 18, 19 or 20 serotypes, including serotypes 3, 5, 6B, 12F, 15B, 18C, 19F and 23F, are respectively conjugated to the carrier protein TTD or a variant thereof, or
   the serotypes include 24 serotypes in which the capsular polysaccharides of 8, 9, 10, 11, 12, 13, 14, 15, 16, 17, 18, 19, 20, 21, 22, 23 or 24 serotypes, including serotypes 3, 5, 6B, 12F, 15B, 18C, 19F and 23F, are respectively conjugated to the carrier protein TTD or a variant thereof,
   more preferably,
   the serotypes include 20 serotypes, wherein the capsular polysaccharides of serotypes 3, 5, 6B, 12F, 15B, 18C, 19F and 23F are respectively conjugated to the carrier protein TTD or a variant thereof; wherein the capsular polysaccharides of serotypes 1, 4, 6A, 7F, 8, 9V, 10A, 11A, 14, 19A, 22F and 23F are respectively conjugated to the carrier protein CRM197, or
   the serotypes include 24 serotypes, wherein the capsular polysaccharides of serotypes 3, 5, 6A, 6B, 9N, 11A, 12F, 15B, 17F, 18C, 19A, 19F, 20, 23F and 33F are respectively conjugated to the carrier protein TTD or a variant thereof; wherein the capsular polysaccharides of serotypes 1, 2, 4, 7F, 8, 9V, 10A, 14 and 22F are respectively conjugated to the carrier protein CRM197, or
   the serotypes include 24 serotypes, wherein the capsular polysaccharides of serotypes 3, 5, 6B, 12F, 15B, 18C, 19F and 23F are respectively conjugated to the carrier protein TTD or a variant thereof; wherein the capsular polysaccharides of serotypes 1, 2, 4, 6A, 7F, 8, 9N, 9V, 10A, 11A, 14, 17F, 19A, 20, 22F, and 33F are respectively conjugated to the carrier protein CRM197.
8. The multivalent immunogenic composition of item 5 or 6, wherein the multivalent immunogenic composition futher has one or more characteristics selected from the group consisting of:
   in the composition, the weight ratio of the capsular polysaccharide from serotype 3, 6B or 12F to any other one capsular polysaccharide is 10:1 to 1:10, such as 5:1 to 1:5,
   the composition is a preparation, and the concentrations of capsular polysaccharides from serotypes 3, 6B and 12F are each independently 1 to 8 µg/dose, and the concentrations of the remaining capsular polysaccharides are each independently 0.5 to 5 µg/dose,
   the composition further includes an adjuvant; preferably, the adjuvant includes one or more selected from the group consisting of: aluminum-based adjuvants, monophosphoryl lipid A, QS21, CpG, MF59, stearoyltyrosine, Freund's adjuvant and other mucosal adjuvants,
   in the composition, the weight ratio of conjugate to adjuvant is 1:10 to 1:2,
   the composition futher contains a surfactant; preferably, the concentration of the surfactant in the composition is 100 to 300 µg/dose,
   the pH of the composition is 5.0 to 7.0.
9. The use of the immunogenic composition according to any one of items 1 to 8 in the preparation of a medicament for inducing an immune response to a pneumococcal capsular polysaccharide conjugate and/or an immune response to tetanus toxin,
   preferably, the medicament is used to prevent or treat pneumococcal infection and/or tetanus toxin infection.
10. An immune composition that results in passive immunity, comprising a bactericidal antibody targeting pneumococci, the antibody being obtained by immunizing a mammal with the immunogenic composition of any one of items 1-8,
   preferably, the bactericidal antibodies are present in serum, gamma globulin fractions or purified antibody preparations.

The present description will be described below in the form of specific examples. It should be understood that these examples are merely illustrative and are not intended to limit the scope of the present description. Unless otherwise specified, the methods and materials used in the examples are conventional materials and methods in the art.

### EXAMPLE

### Experimental Methods

### 1. Preparation of pneumococcal capsular polysaccharide

Pneumococcal bacterial culture\fermentation and polysaccharide preparation is a mature process (US4686102, US5847112). Pneumococcal strains of different serotypes (American Type Culture Collection (ATCC)) were cultured to prepare a bacterial strain library. One tube of Streptococcus pneumoniae strains in the bacterial strain library was inoculated into a shake flask containing soybean culture medium and incubated overnight in a 5-10% CO₂ incubator at 37°C. After the culture was completed, microscopic examination was performed. If the result is normal, the culture fluid was inoculated into a 10 L fermentation tank and fermented at pH 7.0 and at a temperature of 37°C. After the culture was completed, 10% DOC solution was added for inactivation to ensure complete inactivation of pneumococcal bacteria.

The purification of pneumococcal capsular polysaccharide is accomplished by an organic solvent-free method (US Patent 5714354). The preparation and purification process mainly includes: the bacterial inactivated liquid was centrifuged to separate the thallus, the centrifugal supernatant was harvested, and then filtered through a 0.22 µm filter membrane and concentrated through 100 KD membrane cassette ultrafiltration to obtain the pneumonia polysaccharide fermentation concentrate. CTAB solution was added to the pneumonia polysaccharide fermentation concentrate, the above solution was stirred at room temperature, centrifuged at 8000 rpm, and the polysaccharide complex precipitate was collected (for 7F, 14, and 33F polysaccharides, the supernatant was collected). The polysaccharide complex was then dissociated with 0.25-0.5M NaCl solution, and then purified through precipitation, column chromatography, ultrafiltration and other processes to obtain a purified polysaccharide solution. The polysaccharide solution was filtered through a 0.22 µm membrane and stored at low temperature.

The prepared different types of pneumococcal capsular polysaccharides can be used to identify serotypes through specific serum, the chemical structure of the pneumococcal polysaccharides can be analyzed through nuclear magnetic resonance (NMR) spectroscopy, and the content of functional groups (such as rhamnose, uronic acid, O-acetyl and other components) contained in polysaccharides can be determined through chemical color development methods. It was determined that the physical and chemical indicators of the polysaccharide meet the standards set by the European Pharmacopoeia for pneumococcal polysaccharides, and the contents of major impurities such as protein and nucleic acid are less than 1%.

### 2. Preparation of carrier protein TTD

The DNA sequence expressing TTD (SEQ ID NO: 1) was optimized and cloned into the protein expression plasmid pET21 to construct E. coli BL21 (DE3) recombinantly expressing the TTD protein (SEQ ID NO: 2). A single clone of recombinant BL21 (DE3) was picked and cultured in 10 mL of LB (Amp) liquid medium at 37°C and 250 rpm until OD₆₀₀ reaches 0.8. Then 0.1mM IPTG was added and continued culturing for 4 hours at 25°C and 250rpm. The culture medium was centrifuged at 8000 rpm and 4°C to collect the thallus, then the thallus were resuspended in PBS and disrupted. The fractions were centrifuged at 8000 rpm, 4 °C, and the supernatant was collected. The TTD protein is purified through ammonium sulfate precipitation, clarification and filtration, and combined chromatography, with a purity of more than 95%. The molecular weight determined by mass spectrometry was consistent with the theoretical molecular weight, and the homogeneity of the TTD protein was confirmed by molecular sieves. The TTD amino acid sequence is shown below:

KNLDCWVDNEEDIDVILKKSTILNLDINNDIISDISGFNSSVITYPDAQLV
PGINGKAIHLVNNESSEVIVHKAMDIEYNDMFNNFTVSFWLRVPKVSASHL
EQYGTNEYSIISSMKKHSLSIGSGWSVSLKGNNLIWTLKDSAGEVRQITFR
DLPDKFNAYLANKWVFITITNDRLSSANLYINGVLMGSAEITGLGAIREDNNI
TLKLDRCNNNNQYVSIDKFRIFCKALNPKEIEKLYTSYLSITFLRDFWGNPL
RYDTEYYLIPVASSSKDVQLKNITDYMYLTNAPSYTNGKLNIYYRRLYNGLK
FIIKRYTPNNEIDSFVKSGDFIKLYVSYNNNEHIVGYPKDGNAFNNLDRILRV
GYNAPGIPLYKKMEAVKLRDLKTYSVQLKLYDDKNASLGLVGTHNGQIGN
DPNRDILIASNWYFNHLKDKILGCDWYFVPTDEGWTND (SEQ ID NO:2)

### 3. Preparation of non-toxic variant CRM197 protein of diphtheria bacterium

The CRM197 carrier protein is a non-toxic variant of diphtheria toxin (SEQ ID NO: 3), which is non-toxic but retains the immunogenicity of diphtheria toxin. Its fermentation and purification methods are available in published articles and patents (US5614382). Usually, the Corynebacterium diphtheriae strain is inoculated into the culture medium, stirred at 37°C for 20-30 hours, and then removed by centrifugation, the supernatant is retained. After the fermentation supernatant is replaced by ultrafiltration, ammonium sulfate is added to precipitate and the precipitate is collected. The precipitate is then dissolved, the liquid is changed, and the CRM197 protein is gained through column chromatography and purified with a purity of more than 95% for use in the binding reaction.

### 4. Preparation of polysaccharide-protein conjugates

The preparation of polysaccharide-protein conjugates by coupling bacterial polysaccharides with carrier proteins is an effective way to improve the immunogenicity of bacterial polysaccharides. Polysaccharide-protein conjugates are widely used in the preparation of bacterial vaccines, such as Haemophilus influenzae type B polysaccharide PRP-TT conjugate vaccine, and Neisseria meningitis bacteria A, C, Y and W polysaccharide-carrier protein conjugate vaccines. The preparation of polysaccharide-protein conjugates is mainly divided into two steps: hydrolysis or activation of polysaccharides, and polysaccharide-protein conjugation. Polysaccharide activation can be divided into different methods such as CNBr method (US4619828), hydrolysis (US4761283) and sodium periodate oxidation method (US5306492). The activated polysaccharide can then be directly chemically conjugated to the carrier protein (US4356170) or through small molecules linker coupled to the carrier protein.

The preparation of pneumococcal polysaccharide-protein conjugates mainly includes the following steps: polysaccharide treatment, polysaccharide activation and polysaccharide-protein conjugation.Pneumococcal polysaccharide pretreatment: some serotypes can be hydrolyzed by acid or alkali (US5847112A) before oxidation to reduce the molecular weight or improve the oxidative binding efficiency of polysaccharides, such as serotypes 1, 3, 4, 6A, 6B, 8, 10A, 11A, 12F, 15B, 18C, 22F polysaccharides. Some polysaccharides can reduce their molecular size through high-pressure homogenization, such as serotypes 2, 6B, 7F, 8, 10A, 11A, 12F, 14, 15B, 19A, 19F, and 33F.

Pneumococcal polysaccharide activation: polysaccharide activation is performed by adding sodium periodate solution (US4711779). The amount of periodate added is usually 0.05 to 2 equivalents of the polysaccharide and the oxidation time is 12-24 hours. After the polysaccharide is activated, the small molecular substances in the reaction are removed by ultrafiltration and concentration.

Preparation of pneumococcal polysaccharide-protein conjugates: the chemical coupling of polysaccharide-protein is carried out by reductive amination method (US5952454, EP0720485, US4711779). Polysaccharide-protein conjugates are prepared by conjugating serotypes 1, 2, 4, 6A, 7F, 8, 9N, 9V, 10A, 11A, 14, 17F, 19A, 20, 22F and 33F polysaccharides to CRM97; and polysaccharide-protein conjugates are prepared by conjugating serotypes 3, 5, 6B, 12F, 15B, 18C, 19F and 23F polysaccharides to TTD. In the conjugating reaction, the ratio of polysaccharide to carrier protein is controlled 0.5 to 2.5. During the reaction, 1.0 to 2.0 equivalents of sodium cyanoborohydride solution is added, and then 2.0 equivalents of sodium cyanoborohydride solution is added. After the reaction, ultrafiltration liquid exchange or chromatography is used to remove impurities or unreacted substrates in the reaction. Finally, sterile filtration was performed through a 0.22 µm filter, and the polysaccharide-protein conjugate solution was stored at 2 to 8°C. All polysaccharide-protein conjugates have passed quality analysis. The free polysaccharide content is controlled within 20%, the free protein content is controlled within 2%, the polysaccharide-protein ratio is 0.5 to 2.0, and the endotoxin and other impurity contents are controlled within safe and acceptable range.

### 5. Preparation of multivalent pneumococcal polysaccharide-protein conjugate vaccine

There are 24 main pathogenic serotypes of pneumococci, namely 1, 2, 3, 4, 5, 6A, 6B, 7F, 8, 9N, 9V, 10A, 11A, 12F, 14, 15B, 17F, 18C, 19A, 19F, 20, 22F, 23F and 33F. Each polysaccharide-protein monovalent conjugate stock solution was prepared by diluting with a pH 6.0 buffer solution and mixing, then adding aluminum phosphate adjuvant and stirring. The polysaccharide content in various conjugates is 2 to 4 micrograms per milliliter, and the aluminum phosphate adjuvant content is 0.125 to 0.5 mg.

### 6. Evaluation of the immunogenicity of polysaccharide-protein conjugates in animals

**Mouse immunization experimental protocol:** the aluminum phosphate adjuvant was added into the polyvalent vaccine stock solution to prepare an immune antigen. Balb/c mice aged 6-8 weeks were selected for intraperitoneal immunization. There are 8 mice in each group, with each immunization dose is 0.5ml, immunized on days 0, 14, and 21 respectively. Relevant vaccines that have been launched (Pfizer's Prevenar 13, Walvax Biotechnology's Weuphoria) were used as positive controls, and aluminum phosphate adjuvant was used as negative controls. At day 35, blood was obtained and the immunogenicity of polysaccharides of the above immune antigen was evaluated.

**Rabbits immunization experimental protocol:** the aluminum phosphate adjuvant was added into the polyvalent vaccine stock solution to prepare an immune antigen. New Zealand white rabbits of 2.0 to 2.5kg were selected for thigh muscle immunization. There are 8 rabbits in each group and each immunization dose is 0.5ml. At the same time, PCV13 (Prevenar 13) is used as the positive vaccine for two doses of immunization. At day 35, blood was obtained and the immunogenicity of polysaccharides of the above immune antigen was evaluated.

### 7. Evaluating polysaccharide immunogenicity by ELISA

Pneumonia polysaccharide was diluted with coating buffer, coated on a 96-well enzyme plate at 100 µl/well, and incubated at 37°C before washing the plate. After treating the mouse and rabbit antiserum with the adsorbent, firstly the serum was diluted at 1:100, and then diluted at 2.5 times of gradient for 8 gradients, and then added to the enzyme label plate with 50 µl per well, and incubated overnight. After washing the plate, the secondary antibody was diluted at 1:10000 and added to the ELISA plate with 100 µl per well, and the plate was washed after incubating for 2 hours, then 1 mg/ml PNPP-Na chromogenic substrate was added with 100 µl per well, and after incubating for 2 hours, 50 µl/well of 3M NaOH was added to stop the reaction, and the absorbance at a wavelength of 405 nm was recorded. If the ratio of the OD value of the test well to the OD value of the negative well is greater than or equal to 2.1, it is judged as positive, and the antibody titer of each serum is defined as the maximum dilution fold where the ratio shows positive. The geometric mean of the antibody titer of each group of immunized animals was calculated, using T-test to analyze the statistical significance among different groups.

### Example 1: Comparison of the immunogenicity of serotype 5 polysaccharide-CRM197 conjugate and pneumococcal polysaccharide-TTD conjugate in mice

The 5PS-CRM197 conjugate, 5PS-TTD conjugate and aluminum phosphate adjuvant were mixed, then the BALB/c mice (5 mice/group) were immunized with the above mixture at 2 µg/dose on Day 0, 14, and 28, respectively. Blood were collected at Day 21 and 35 to detect antibody titers.

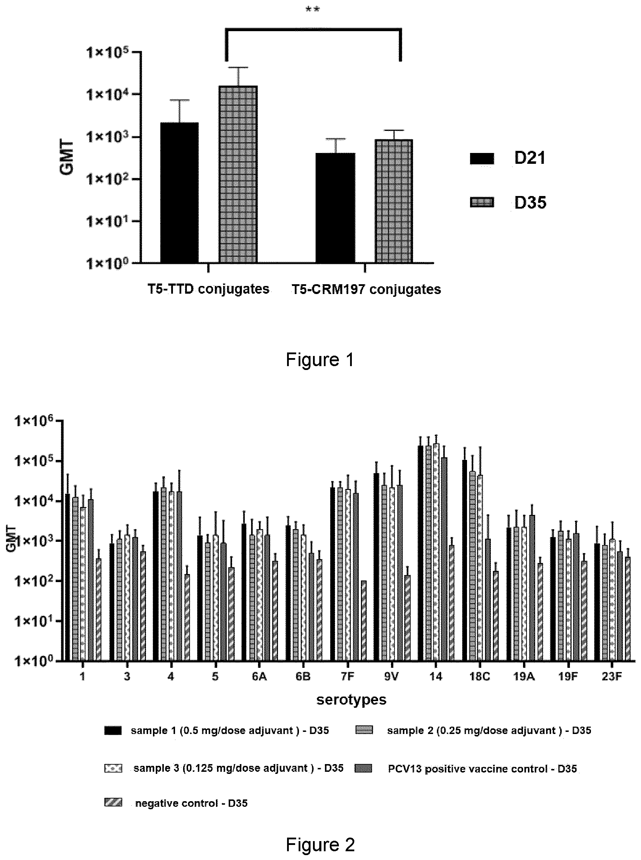

The results show (Figure 1) that the D35 titer level of the conjugate of CRM197 carrier is 866.43, and the D35 titer level of the conjugate of TTD carrier is 16260.67. The immunogenicity of the polysaccharide conjugate using TTD as the carrier is higher than that using CRM197 as the carrier, and the difference is analyzed as significant (P=0.0079) by Mann Whitney test **.Example 2: Comparison of the immunogenicity of 24-valent pneumococcal polysaccharide conjugate vaccine in mice at different adjuvant doses**

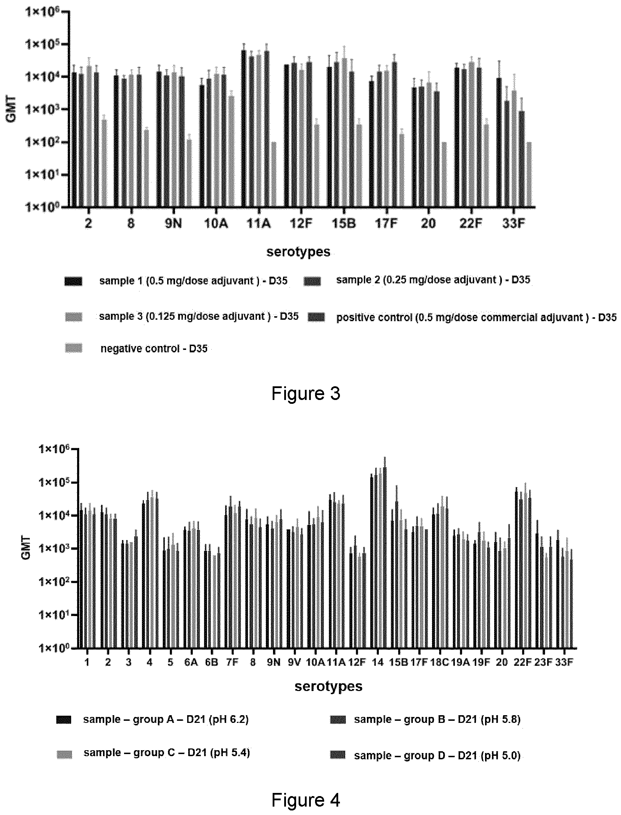

The immunogenicity differences of the 24-valent pneumonia polysaccharide conjugate vaccine with aluminum phosphate adjuvant dosages of 0.5 mg/dose, 0.25 mg/dose, and 0.125 mg/dose, respectively, is evaluated in BALB/c mice. BALB/c female mice aged 6 to 8 weeks were randomly divided into groups, 8 mice/group, and were immunized with 0.5 ml/dose on Days 0, 14, and 28 respectively. Blood was collected on Day 35 to detect antibody titer levels, and the originality data are shown in Figures 2 and 3.

The results show that the immunogenicity of the 24-valent pneumococcal polysaccharide conjugate vaccine against 13 serotypes (serotypes included in the positive vaccine) at different adjuvant doses is equivalent to that of the launched 13-valent conjugate vaccine. For some serotypes, the 24-valent pneumococcal polysaccharide conjugate vaccine show better antibody titers than the 13-valent conjugate vaccine, such as serotype 6B, 18C, 23F. At the same time, for 11 serotypes other than the 13 serotypes, good immunogenicity are also achieved.

### Example 3: Comparison of the immunogenicity of 24-valent pneumococcal polysaccharide conjugate vaccine in mice with different pH conditions

The immunogenicity of 24-valent pneumococcal polysaccharide conjugate vaccines at different were evaluated in BALB/c mice. The mice were randomly divided into groups, with 8 mice/group, and the 24-valent pneumonia polysaccharide conjugate vaccine was evaluated in 6-to 8-week-old BALB/c female mice. In the test, a total of two immunizations were conducted on D0 and D14, one dose per time, and serum was collected on D0 and D21, and the polysaccharide antibody titer level in the serum was detected by ELISA.

Fot 24-valent pneumococcal polysaccharide conjugate vaccine in the pH range of 5.0 to 6.2, serum antibody titers were compared one week (D21) after immunization with two doses (Figure 4). The result illustrate that, after immunization with 24-valent pneumococcal polysaccharide conjugate vaccine with the pH range of 5.0 to 6.2, there is no significant difference in the antibody titer for each serotype between groups (P>0.05), and all vaccines induce good immunogenicity.

### Example 4: Comparison of the immunogenicity of 24-valent pneumococcal polysaccharide conjugate vaccine and positive vaccine in mice

There are two types of 13-valent pneumonia conjugate vaccines currently on the market, one is Prevenar 13, produced by Pfizer of the United States, using CRM197 as the carrier protein, and the other is Weuphoria, produced by Walvax Biotechnology, using tetanus toxoid (TT) as the carrier protein.

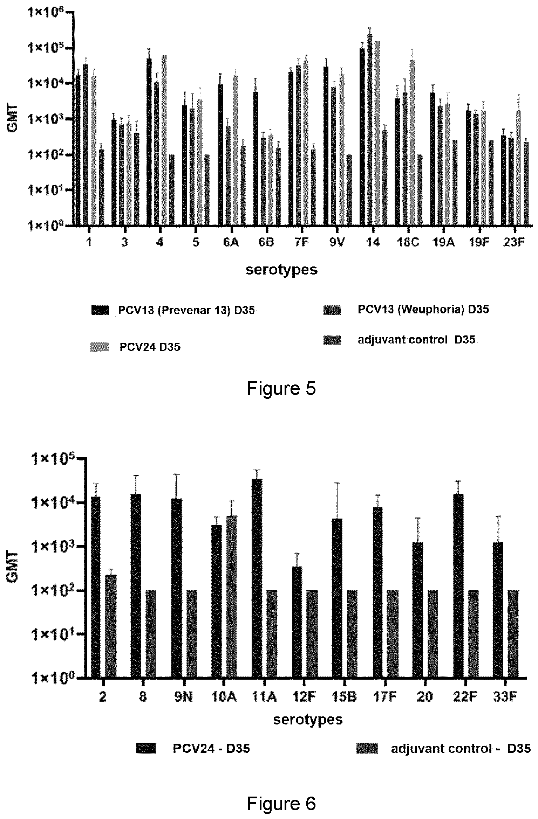

After immunizing BALB/c mice with the 24-valent pneumonia conjugate vaccine, Prevenar 13, Weuphoria and the adjuvant control group on days 0, 14 and 28 respectively, the immunogenicity on day 35 was evaluated, and the results are shown in Figure 5 and Figure 6.

The results show that:
(1) Comparing the 24-valent pneumococcal conjugate vaccine with Prevenar 13 (based on CRM197 carrier protein), the immunogenicity against serotype 18C is significantly higher than that of Prevenar 13. In addition, the antibody titers for serotype 5, 6A, 7F, and 23F are also higher than the control group.
(2) Comparing the 24-valent pneumococcal conjugate vaccine with the Weuphoria (based on TT carrier protein), the D35 serum antibody titers were tested by the non-parametric Kruskal-Wallis test and there were significant differences in serotype 4, 6A and 23F, wherein the 24-valent pneumococcal conjugate vaccine shows higher immunogenicity than Weuphoria. In addition, the antibody titers for serotype 5, 7F, and 9V are also higher than those in the control group.
(3) For non-PCV13 vaccine serotypes, the 24-valent pneumococcal conjugate vaccine can also induce better immunogenicity (Figure 6). The antibody titers of the 24-valent pneumococcal conjugate vaccine (PCV24) and the adjuvant control group at Day 35 are analyzed by the Mann-Whitney test. There are significant differences in serotypes 2, 8, 9N, 11A, 15B, 17F, 20, and 33F. (P<0.05), indicating that the PCV24 vaccine has better immunogenicity against serotypes other than PCV13. There is no significant difference between the serotype 10A vaccine group and the adjuvant control group (P=0.3068), which may be due to the higher background antibody titer level of serotype 10A in experimental mice.

### Example 5: Comparison of the immunogenicity of 24-valent pneumococcal polysaccharide conjugate vaccine and positive vaccine in rabbits

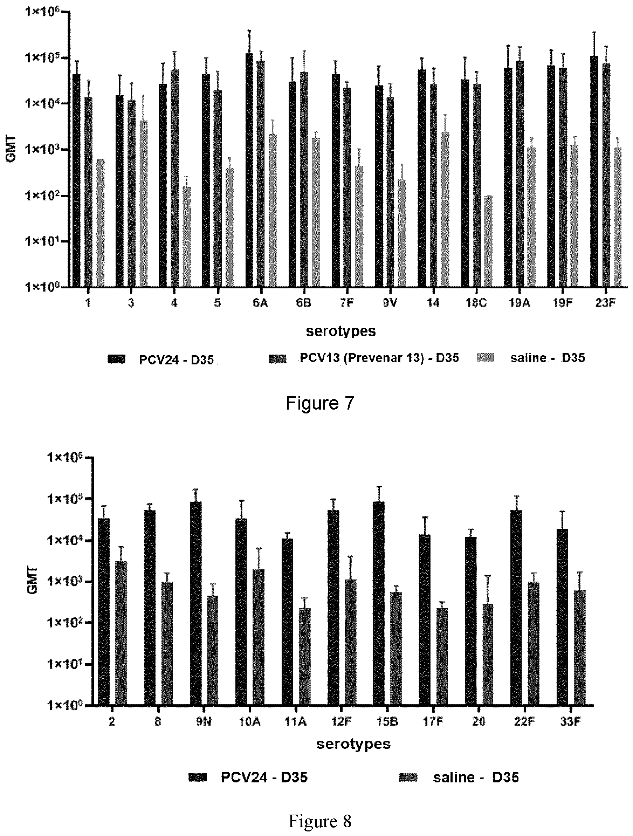

PCV24 pneumococcal polysaccharide conjugate vaccine is prepared to immunize rabbits (8/group), using PCV13 (Prevenar 13) as the positive control and physiological saline as the negative control. After the first immunization, futher immunization is conducted after three weeks. Two weeks after the second immunization (D35), blood was collected to detect the antibody titers of various serotype of polysaccharides in the serum (Figure 7 and Figure 8).

Thirteen serotypes were analyzed by T test after two doses of PCV24 immunization and compared with PCV13 (Prevenar 13) (Figure 7), indicating that the immunogenicity can reach the level of the lanuched vaccines. For eleven serotypes other than the 13 serotypes (Figure 8), good immunogenicity were also achieved.

### Example 6: Comparison of the immunogenicity of dual-carrier and single-carrier 24-valent pneumococcal polysaccharide conjugate vaccine

In order to compare the immunogenicity difference between the 24-valent pneumococcal polysaccharide conjugate vaccine using two carrier proteins and that using one carrier protein, two different 24-valent pneumococcal polysaccharide conjugate vaccines were prepared. For Group A vaccine, 8 serotypes,which are serotype 3, 5, 6B, 12F, 15B, 18C, 19F, and 23F, use TTD as the carrier protein, and the other 16 serotypes use CRM197 as the carrier protein; for Group B vaccine, all 24 serotypes use TTD as the carrier protein .

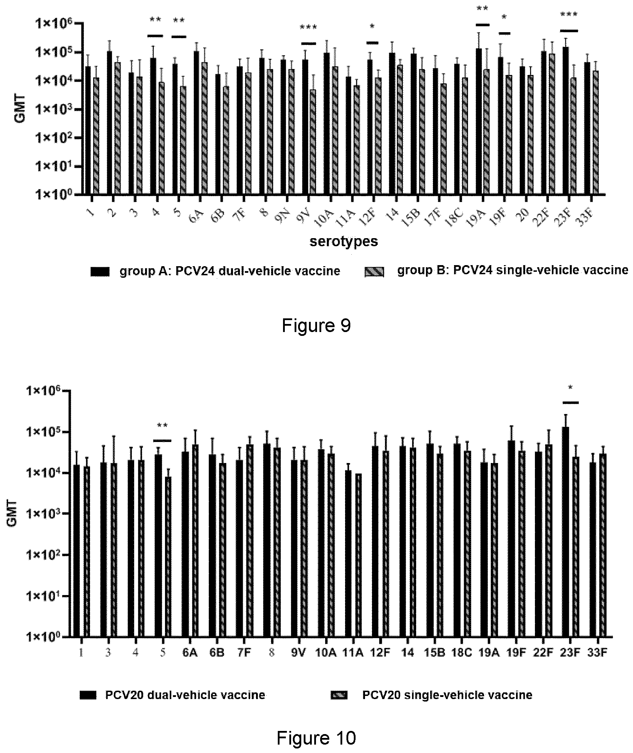

The above two groups of vaccines are used to immunize New Zealand white rabbits respectively, with 8 rabbits in each group, and the rabbits were immunized twice in total with an interval of three weeks. Serum was collected before immunization and two weeks after the second immunization to detect the titer levels of various types of polysaccharide antibodies. The vaccine titers of the two groups two weeks after the second immunization were converted into logarithms and statistically analyzed using Multiple t tests and the results are shown in Figure 9.

As can be seen from Figure 9, among all 24 serotypes, the dual-carrier vaccine group can induce higher antibody titers than the single-carrier vaccine group, and the induced antibody titers of 7 serotypes (serotype 4, 5, 9V, 12F, 19A, 19F, 23F) are significantly different (P<0.05). It shows that the dual-carrier vaccine can avoid the immunosuppressive effect caused by a single carrier.

### Example 7: Comparison of the immunogenicity of dual-carrier and single-carrier 20-valent pneumococcal polysaccharide conjugate vaccine

Two different 20-valent (serotype 1, 3, 4, 5, 6A, 6B, 7F, 8, 9V, 10A, 11A, 12F, 14, 15B, 18C, 19A, 19F, 22F, 23F, 33F) pneumococcal polysaccharide conjugate vaccine were prepared. For Group A vaccine, 8 serotypes, which are 3, 5, 6B, 12F, 15B, 18C, 19F, and 23F, use TTD as the carrier protein, , and the other 12 serotypes use CRM197 as carrier protein; for Group B vaccine, all 20 serotypes use TTD as the carrier protein.

The above two groups of vaccines were used to immunize New Zealand white rabbits respectively, with 8 rabbits in each group, and the rabbits were immunized twice in total with an interval of three weeks. Serum was collected before immunization and two weeks after the second immunization to detect the titer levels of various types of polysaccharide antibodies. The vaccine titers of the two groups two weeks after the second immunization were converted into logarithms and statistically analyzed using Multiple t tests. The results are shown in Figure 10.

The results shows that, for the 8 serotypes (serotype 3, 5, 6B, 12F, 15B, 18C, 19F, 23F) in the dual-carrier vaccine group using TTD carriers, the antibody titer levels are all higher than those in the single-carrier vaccine group, with a significant difference in antibody titers for serotypes 5 and serotypes 23F (P<0.05).

For 16 of the 20 serotypes in the dual-carrier vaccine (except serotype 6A, 7F, 22F, and 33F), the titer levels are higher than those in the single-carrier vaccine group.

### Example 8: Comparison of the immunogenicity of dual-carrier and single-carrier 24-valent pneumococcal polysaccharide conjugate vaccine

Two different 24-valent (serotype 1, 2, 3, 4, 5, 6A, 6B, 7F, 8, 9N, 9V, 10A, 11A, 12F, 14, 15B, 17F, 18C, 19A, 19F, 20, 22F, 23F and 33F) pneumococcal polysaccharide conjugate vaccine were prepared. For Group A vaccine, 15 serotypes, which are 3, 5, 6A, 6B, 9N, 11A, 12F, 15B, 17F, 18C, 19A, 19F, 20, 23F and 33F, use TTD as the carrier protein, and the other 9 serotypes use CRM197 as the carrier protein; for Group B vaccine, all 24 serotypes use TTD carrier protein as the carrier protein.

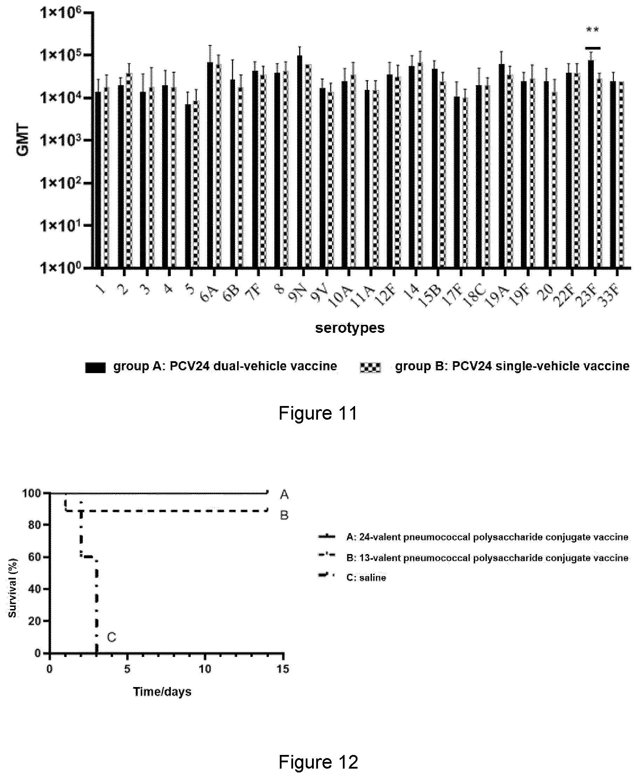

The above two groups of vaccines were used to immunize New Zealand white rabbits respectively, with 8 rabbits in each group, and the rabbits were immunized twice in total with an interval of three weeks. Serum was collected before immunization and two weeks after the second immunization to detect the titer levels of various types of polysaccharide antibodies. The vaccine titers of the two groups two weeks after the second immunization were converted into logarithms and statistically analyzed using Multiple t tests. The results are shown in Figure 11.

The results shows that, for the 15 serotypes (serotype 3, 5, 6A, 6B, 9N, 11A, 12F, 15B, 17F, 18C, 19A, 19F, 20, 23F, 33F) in the dual-carrier vaccine group using TTD carriers, 12 serotypes show higher antibody titer than those of the single-carrier vaccine group, with a significant difference in antibody titer for serotypes 23F (P<0.05).

For 16 of the 24 serotypes in the dual-carrier vaccine (except serotype 1, 2, 3, 4, 8, 10A, 14, 19F), the titer levels are higher than those in the single-carrier vaccine group.

**Example 9: Protection test of 24-valent pneumococcal polysaccharide conjugate vaccine against serotype 3 pneumococci in BALB/c mice** In BALB/c mice, PCV13 (Walvax Biotechnology, Weuphoria) polysaccharide conjugate vaccine was used as the positive control and normal saline was used as the negative control. Two weeks after three doses of intraperitoneal immunization, BALB/c mice were intranasally infected with 2 × 10⁵ CFU of serotype 3 streptococcus pneumoniae. The survival rate within two weeks after challenge was observed, and the protection of the 24-valent pneumococcal polysaccharide conjugate vaccine against serotype 3 pneumococcal was analyzed.

The results (Figure 12) show that the antibodies induced by the 24-valent pneumococcal polysaccharide conjugate vaccine have 100% immune protection rate, which is better than the lanuched 13-valent pneumococcal polysaccharide conjugate vaccine having a protection rate of 89.9%.

### Example 9: Protection test of 24-valent pneumococcal polysaccharide conjugate vaccine against serotype 22 pneumococci in BALB/c mice

The experiment use 23-valent pneumococcal polysaccharide vaccine (PPV23) as the positive control and normal saline as the negative control. Three weeks after one dose of intraperitoneal immunization, BALB/c mice were intraperitoneally infected with 1×10⁸ CFU of serotype 22F streptococcus pneumoniae. The protection of the 24-valent pneumococcal polysaccharide conjugate vaccine against serotype 22 pneumococcal was analyzed by assessing the survival of mice after challenge.

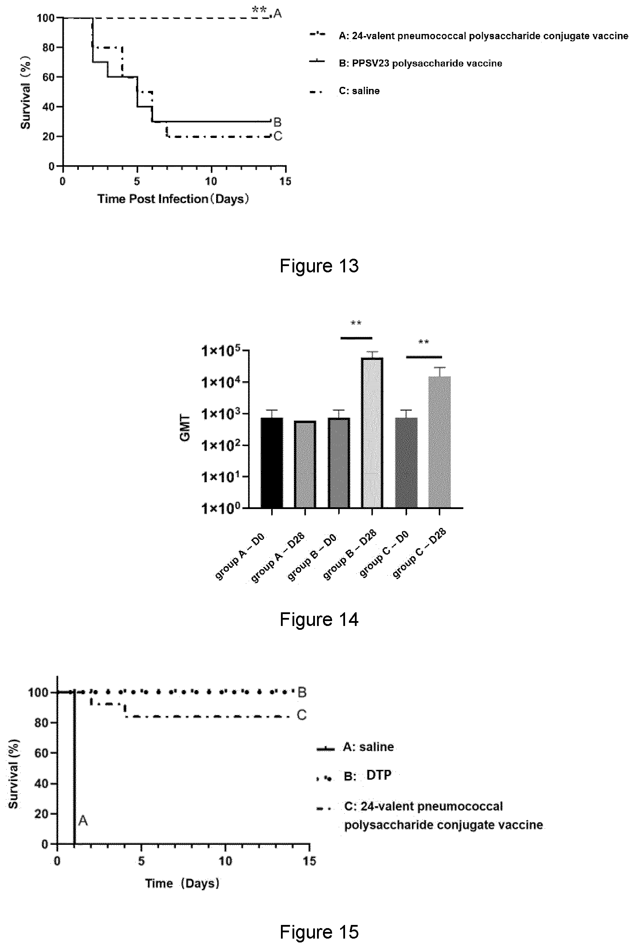

The results (Figure 13) show that the group of 24-valent vaccine has a survival rate of 100%, the group of control PPV23 polysaccharide vaccine has a survival rate of 30%, and the group of physiological saline has a survival rate of 20%. Based on Log-rank (Mantel-Cox) test, the 24-valent vaccine has a significant difference compared with the control, P < 0.05 (P value is 0.0027).

In summary, compared with the PPV23 polysaccharide vaccine, the 24-valent pneumococcal polysaccharide conjugate vaccine has a significant protective effect against serotype 22F pneumococcal.

### Example 10: Protection test of 24-valent pneumococcal polysaccharide conjugate vaccine against tetanus toxin

The 24-valent pneumococcal polysaccharide conjugate vaccine contains TTD carrier protein, and the effectiveness of the vaccine was evaluated through a tetanus toxin challenge protection test in mice.

In BALB/c mice, 24-valent pneumococcal polysaccharide conjugate vaccine was injected intraperitoneally on days 0 and 14, with DTP (Diphtheria-Tetanus-Pertussis) vaccine as a positive control and normal saline as a negative control. On Day 28, blood was collected to detect the antibody titer level against tetanus toxoid (TT) in the serum (Figure 14, group A is normal saline; group B is DTP vaccine; group C is 24-valent pneumococcal polysaccharide conjugate vaccine). Tetanus toxin (20LD50) was intraperitoneally injected, and the survival of the mice within two weeks were observed (Figure 15).

As can be seen from Figure 14, the TT antibody titer levels in groups B, C, and D are significantly increased after two doses of immunization compared with those before immunization, P<0.05. The TT antibody titer in group B is higher than that in group C, possibly because the DTP vaccine is composed of TT, and the 24-valent pneumococcal polysaccharide conjugate vaccine use TTD.

It shows in Figure 15 that in the normal saline group, all the mice died on the first day after the tetanus toxin intraperitoneal infection; the protection rate of the 24-valent pneumococcal polysaccharide conjugate vaccine is 80%; and the protection rate of the DTP vaccine control group was 100%. This shows that the 24-valent pneumococcal polysaccharide conjugate vaccine has a protective effect on tetanus toxin infection, and its effect is significantly better than the negative control.

## Claims

1. An immunogenic composition containing capsular polysaccharides from different serotypes of Streptococcus pneumonia and a vehicle, the serotypes includes at least 2, 8, 9N, 10A, 11A, 12F, 15B, 17F, 20, 22F and 33F,
preferably, the serotypes include 8, 9, 10, 11, 12, 13, 14, 15, 16, 17, 18, 19, 20, 21, 22, 23, 24 serotypes selected from the following: 1 , 2, 3, 4, 5, 6A, 6B, 7F, 8, 9N, 9V, 10A, 11A, 12F, 14, 15B, 17F, 18C, 19A, 19F, 20, 22F, 23F and 33F, and the serotypes include at least 2, 8, 9N, 10A, 11A, 12F, 15B, 17F, 20, 22F and 33F.

2. The immunogenic composition of claim 1, wherein the serotypes include the following 20 serotypes: 1, 3, 4, 5, 6A, 6B, 7F, 8, 9V, 10A, 11A, 12F, 14, 15B, 18C, 19A, 19F, 22F, 23F, 33F, or the following 24 serotypes: 1, 2, 3, 4, 5, 6A, 6B, 7F, 8, 9N, 9V, 10A, 11A, 12F, 14, 15B, 17F, 18C, 19A, 19F, 20, 22F, 23F and 33F,
preferably, the vehicle is selected from one or more of saline, Ringer's solution and phosphate buffered saline.

3. The immunogenic composition of claim 1 or 2, wherein the immunogenic composition further contains an adjuvant,
preferably, the adjuvant includes one or more selected from aluminum-based adjuvants, monophosphoryl lipid A, QS21, CpG, MF59, stearoyltyrosine, Freund's adjuvant and other mucosal adjuvants.

4. The immunogenic composition of claim 1 or 2, wherein the weight ratio of the capsular polysaccharide from serotype 3, 6B or 12F to any other one capsular polysaccharide is 10:1 to 1:10, for example, 5:1 to 1:5,
preferably, the composition is a preparation, and the concentrations of capsular polysaccharides from serotypes 3, 6B and 12F are each independently 1 to 8 *µ* g/dose, and the concentrations of the remaining capsular polysaccharides are each independently 0.5 to 5 *µ* g/dose.

5. A multivalent immunogenic composition comprising a plurality of polysaccharide-protein conjugates and pharmaceutically acceptable excipients, wherein each polysaccharide-protein conjugate contains capsular polysaccharide from different serotypes of Streptococcus pneumonia conjugated to a carrier protein,
preferably, the carrier protein contains at least two carrier proteins,
preferably, the serotypes include at least 2, 8, 9N, 10A, 11A, 12F, 15B, 17F, 20, 22F and 33F; more preferably, the serotypes include the following 20 serotypes: 1, 3, 4, 5, 6A, 6B, 7F, 8, 9V, 10A, 11A, 12F, 14, 15B, 18C, 19A, 19F, 22F, 23F, 33F, or the following 24 serotypes: 1, 2, 3, 4 , 5, 6A, 6B, 7F, 8, 9N, 9V, 10A, 11A, 12F, 14, 15B, 17F, 18C, 19A, 19F, 20, 22F, 23F and 33F.

6. The multivalent immunogenic composition of claim 5, wherein the carrier protein includes (1) CRM197 and (2) TTD or a variant thereof,
preferably, the TTD is the C-terminal domain of TT,
more preferably, the TTD has the sequence shown in SEQ ID NO.2, and the TTD variant has a sequence with at least 90% sequence identity to SEQ ID NO:2.

7. The multivalent immunogenic composition of claim 5 or 6, wherein, for the polysaccharide protein conjugate, at least one or more or all of the capsular polysaccharides from serotypes 3, 5, 6B, 12F, 15B, 18C, 19F and 23F are respectively conjugated to the carrier protein TTD or a variant thereof,
preferably,
the serotypes include 20 serotypes in which the capsular polysaccharides of 8, 9, 10, 11, 12, 13, 14, 15, 16, 17, 18, 19 or 20 serotypes, including serotypes 3, 5, 6B, 12F, 15B, 18C, 19F and 23F, are respectively conjugated to the carrier protein TTD or a variant thereof, or
the serotypes include 24 serotypes in which the capsular polysaccharides of 8, 9, 10, 11, 12, 13, 14, 15, 16, 17, 18, 19, 20, 21, 22, 23 or 24 serotypes, including serotypes 3, 5, 6B, 12F, 15B, 18C, 19F and 23F, are respectively conjugated to the carrier protein TTD or a variant thereof,
more preferably,
the serotypes include 20 serotypes, wherein the capsular polysaccharides of serotypes 3, 5, 6B, 12F, 15B, 18C, 19F and 23F are respectively conjugated to the carrier protein TTD or a variant thereof; wherein the capsular polysaccharides of serotypes 1, 4, 6A, 7F, 8, 9V, 10A, 11A, 14, 19A, 22F and 23F are respectively conjugated to the carrier protein CRM197, or
the serotypes include 24 serotypes, wherein the capsular polysaccharides of serotypes 3, 5, 6A, 6B, 9N, 11A, 12F, 15B, 17F, 18C, 19A, 19F, 20, 23F and 33F are respectively conjugated to the carrier protein TTD or a variant thereof; wherein the capsular polysaccharides of serotypes 1, 2, 4, 7F, 8, 9V, 10A, 14 and 22F are respectively conjugated to the carrier protein CRM197, or
the serotypes include 24 serotypes, wherein the capsular polysaccharides of serotypes 3, 5, 6B, 12F, 15B, 18C, 19F and 23F are respectively conjugated to the carrier protein TTD or a variant thereof; wherein the capsular polysaccharides of serotypes 1, 2, 4, 6A, 7F, 8, 9N, 9V, 10A, 11A, 14, 17F, 19A, 20, 22F, and 33F are respectively conjugated to the carrier protein CRM197.

8. The multivalent immunogenic composition of claim 5 or 6, wherein the multivalent immunogenic composition futher has one or more characteristics selected from the group consisting of:
in the composition, the weight ratio of the capsular polysaccharide from serotype 3, 6B or 12F to any other one capsular polysaccharide is 10:1 to 1:10, such as 5:1 to 1:5,
the composition is a preparation, and the concentrations of capsular polysaccharides from serotypes 3, 6B and 12F are each independently 1 to 8 µg/dose, and the concentrations of the remaining capsular polysaccharides are each independently 0.5 to 5 µg/dose,
the composition further includes an adjuvant; preferably, the adjuvant includes one or more selected from aluminum-based adjuvants, monophosphoryl lipid A, QS21, CpG, MF59, stearoyltyrosine, Freund's adjuvant and other mucosal adjuvants,
in the composition, the weight ratio of conjugate to adjuvant is 1:10 to 1:2,
the composition further contains a surfactant; preferably, the concentration of the surfactant in the composition is 100 to 300 µg/dose,
the pH of the composition is 5.0 to 7.0.

9. The use of the immunogenic composition according to any one of claims 1 to 8 in the preparation of a medicament for inducing an immune response to a pneumococcal capsular polysaccharide conjugate and/or an immune response to tetanus toxin,
preferably, the medicament is used to prevent or treat pneumococcal infection and/or tetanus toxin infection.

10. An immune composition that results in passive immunity, comprising a bactericidal antibody targeting pneumococci, the antibody being obtained by immunizing a mammal with the immunogenic composition of any one of claims 1-8,
preferably, the bactericidal antibodies are present in serum, gamma globulin fractions or purified antibody preparations.
